# EUROPÄISCHE PATENTSCHRIFT

(11) **EP 1 859 749 B1**
(45) Veröffentlichungstag und Bekanntmachung des Hinweises auf die Patenterteilung: **24.06.2009**
(21) Anmeldenummer: 07106690.6
(22) Anmeldetag: 23.04.2007
(51) Int. Cl.: A61B 17/16

(54) **Druckgasbetätigtes chirurgisches Instrument**
Surgical instrument actuated by gas pressure
Instrument chirurgical activée par gaz sous pression

(30) Priorität: 27.05.2006 DE 102006024760
(43) Veröffentlichungstag der Anmeldung: 28.11.2007
(73) Patentinhaber: Aesculap AG, 78532 Tuttlingen (DE)
(72) Erfinder: Scholten, Thomas, 78532, Tuttlingen (DE); Mayenberger, Rupert, 78239, Rielasingen (DE)
(74) Vertreter: Hoeger, Stellrecht & Partner Patentanwälte

(56) Entgegenhaltungen:
- US-A- 3 815 476
- US-A- 3 837 555
- US-A1- 2004 230 157
- US-A1- 2006 069 395

## Beschreibung

Die Erfindung betrifft ein chirurgisches Instrument mit einem druckgasbetätigten Werkzeug und mit einer Aufnahme für eine Druckgaspatrone sowie einem druckdichten Anschluss zur Verbindung der Druckgaspatrone mit dem Werkzeug gemäß dem Oberbegriff des Anspruchs 1.

Chirurgische Instrumente, beispielsweise Zangen, Scheren, Greifinstrumente oder dergleichen, arbeiten häufig mit Werkzeugen, die bewegt werden müssen. Dies wird üblicherweise durch manuelle Antriebe über ein Griffteil erreicht. Diese Art der Betätigung kann sehr ermüden, und daher ist es bekannt, die Antriebskräfte durch ein Druckgas bereitzustellen.

Es ist möglich, das Druckgas, beispielsweise Pressluft, durch Schlauchleitungen dem Instrument zuzuführen, dies ist jedoch umständlich und kann bei dem Einsatz des Instrumentes hinderlich sein. Es ist weiterhin bekannt, Druckgas in Druckgaspatronen bereitzustellen, die in das Instrument eingesetzt und dort mit den durch das Druckgas zu bewegenden Teilen verbunden werden. Um dabei einen dichten Anschluss der Druckgaspatronen an die jeweiligen Anschlusselemente zu erreichen, ist eine sehr genaue Justierung notwendig, beispielsweise werden Druckgaspatronen dieser Art im Betrieb mit Feingewinden eingeschraubt. Dies ist zeitaufwendig und umständlich. In der US-A-3,837,555 ist ein chirurgisches Instrument beschrieben, das mit einer Druckgaspatrone versehen ist, diese Druckgaspatrone kann durch einen Anstechdorn aufgestochen werden und liegt abgedichtet an dem Einstechdorn an. Eine ähnliche Konstruktion ist in der US 2006/0069395 A1 beschrieben.

Es ist Aufgabe der Erfindung, ein gattungsgemäßes chirurgisches Instrument so auszubilden, dass die Abdichtung zwischen Druckgaspatrone einerseits und dem Instrument andererseits verbessert wird, und insbesondere Fertigungs- und Positionierungstoleranzen ausgeglichen werden, die zu Undichtigkeiten führen könnten.

Diese Aufgabe wird bei einem chirurgischen Instrument der eingangs beschriebenen Art erfindungsgemäß dadurch gelöst, dass die Ringdichtung durch eine Feder gegen die Druckgaspatrone gedrückt wird. Diese federbelastete Ringdichtung legt sich an die Oberseite der Druckgaspatrone an, wenn diese gegen den Rohrstutzen verschoben wird, und dichtet den Kontaktbereich zwischen Rohrstutzen und Druckgaspatrone ab.

Der Verschiebeweg der Ringdichtung kann dabei in Kraftrichtung der Feder durch einen Anschlag begrenzt sein.

Insbesondere ist dieser Anschlag so positioniert, dass die Ringdichtung über den Rohrstutzen vorsteht, so dass die Druckgaspatrone vor dem Kontakt mit dem Rohrstutzen an der Ringdichtung zur Anlage kommt und dieser Bereich daher abgedichtet ist, bevor die Druckgaspatrone geöffnet wird.

Bei einer bevorzugten Ausführungsform ist die Ringdichtung an einer den Rohrstutzen umgebenden Manschette angeordnet, die vorzugsweise durch eine Feder gegen die Druckgaspatrone gedrückt ist.

Der Verschiebeweg der Manschette in Kraftrichtung der Feder wird durch einen Anschlag begrenzt.

Dieser Anschlag kann am Rohrstutzen angeordnet sein.

Gemäß einer besonders bevorzugten Ausführungsform ist vorgesehen, daß der Rohrstutzen und die ihn umgebende Manschette in einer Druckkammer angeordnet sind und daß die Manschette in der Druckkammer in Längsrichtung des Rohrstutzens mittels einer Dichtung abgedichtet verschieblich gelagert ist.

Dabei ist es günstig, wenn der Strömungskanal des Rohrstutzens auf der der Druckgaspatrone abgewandten Seite der Dichtung in die Druckkammer einmündet und wenn die Druckkammer einen zu dem druckgasbetätigten Werkzeug führenden Auslaß aufweist. Auf diese Weise wird die Manschette von gegenüberliegenden Seiten von dem aus der Druckgaspatrone austretenden Druckgas beaufschlagt, so daß Verschiebekräfte auf die Manschette ausgeübt werden.

Wenn gemäß einer bevorzugten Ausführungsform die Querschnittsfläche der Manschette größer ist als die von der Ringdichtung zwischen Manschette und Druckgaspatrone umgebene Fläche, ist die von den Druckgaskräften auf die Manschette ausgeübte Kraft gegen die Druckgaspatrone gerichtet und drückt die Ringdichtung mit einer großen Kraft gegen die Druckgaspatrone, d.h. diese Ausgestaltung führt zu einer besonders zuverlässigen Abdichtung.

Wenn die Ringdichtung durch eine Feder gegen die Druckgaspatrone gedrückt wird, wird die Anpreßkraft, die durch die Feder erzeugt wird, durch diese resultierende Druckgaskraft noch erhöht, so daß es nicht notwendig ist, besonders starke Federn zu verwenden, die die Baugröße heraufsetzen würden.

Die nachfolgende Beschreibung bevorzugter Ausführungsformen der Erfindung dient im Zusammenhang mit der Zeichnung der näheren Erläuterung. Es zeigen:
- Figur 1:: eine perspektivische Ansicht des Handgriffs eines chirurgischen Instrumentes mit einer Aufnahme für eine Druckgaspatrone in der Einsetzposition mit ausgeschwenktem Schwenkhebel;

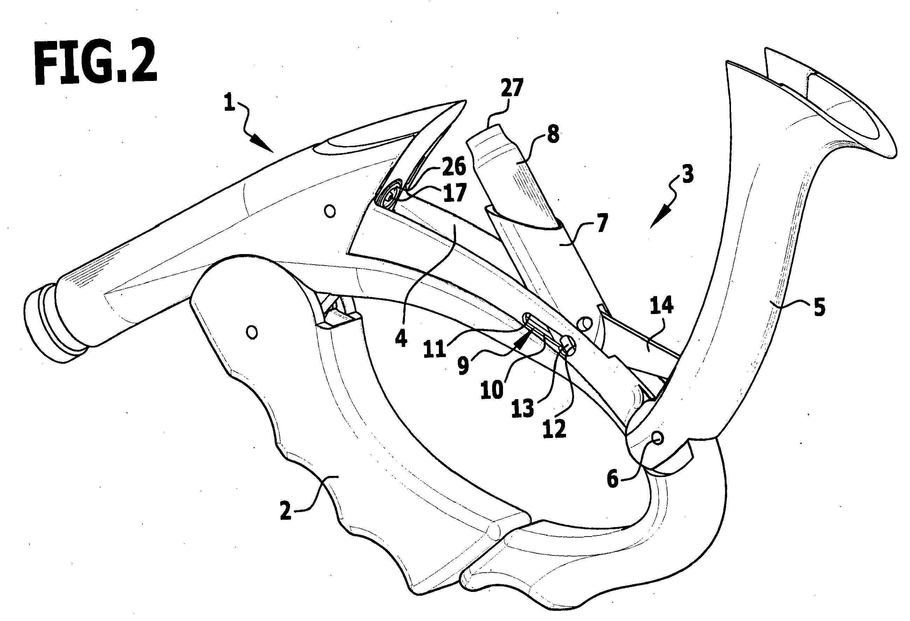

- Figur 2:: eine Ansicht ähnlich Figur 1 beim Beginn der Verschwenkbewegung der Aufnahme in Richtung auf die Anschlußposition;

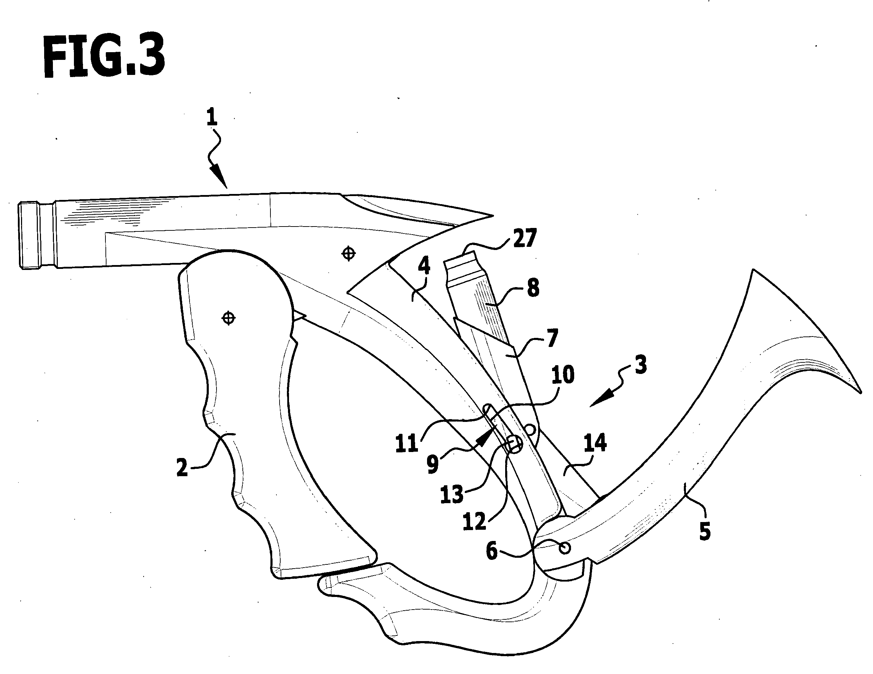

- Figur 3:: eine Seitenansicht des Handgriffs der Figur 2 in einer noch weiter eingeschwenkten Lage der Aufnahme;

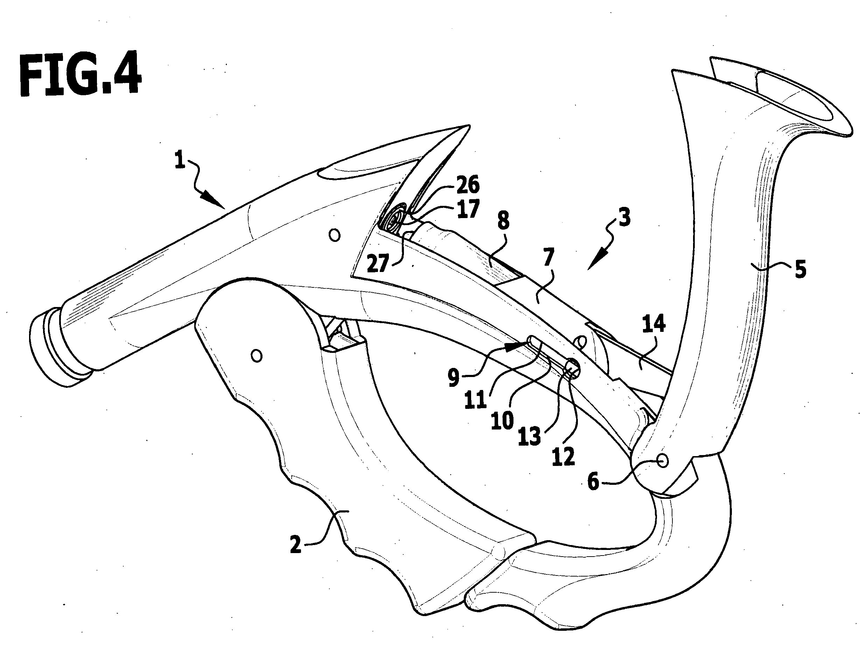

- Figur 4:: eine Ansicht ähnlich Figur 2 mit vollständig eingeschwenkter Aufnahme vor dem Vorschieben der Aufnahme in die Anschlußposition;

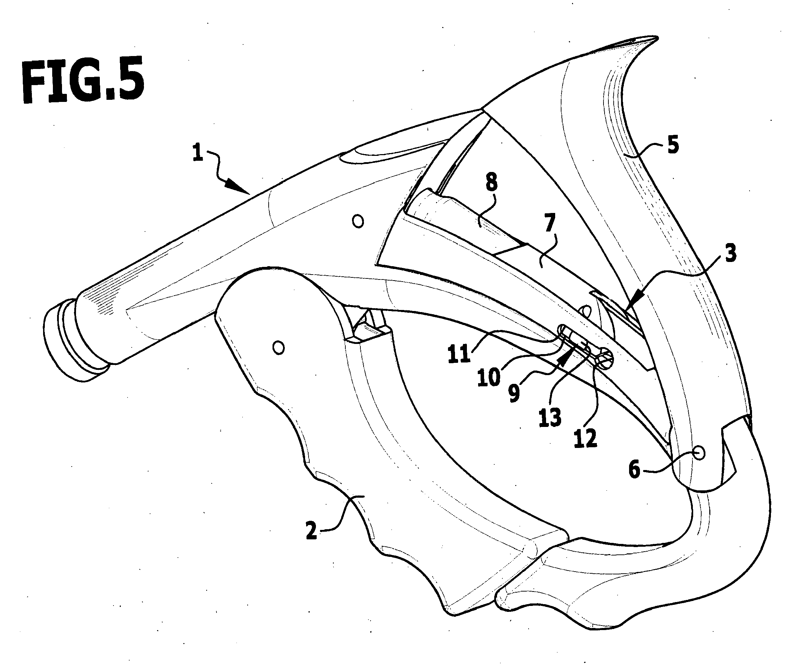

- Figur 5:: eine Ansicht ähnlich Figur 4 mit in Längsrichtung in die Anschlußposition vorgeschobener Aufnahme;

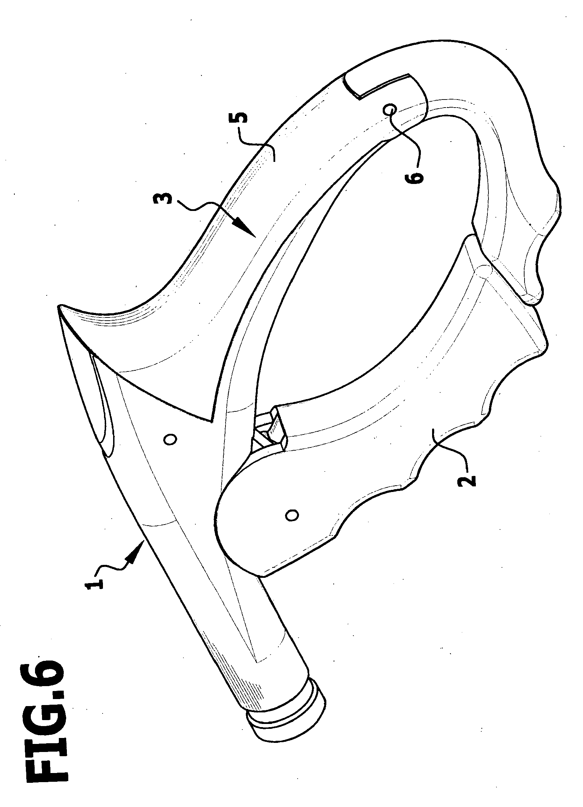

- Figur 6:: eine Ansicht ähnlich Figur 5 mit vollständig eingeschwenktem Schwenkhebel;

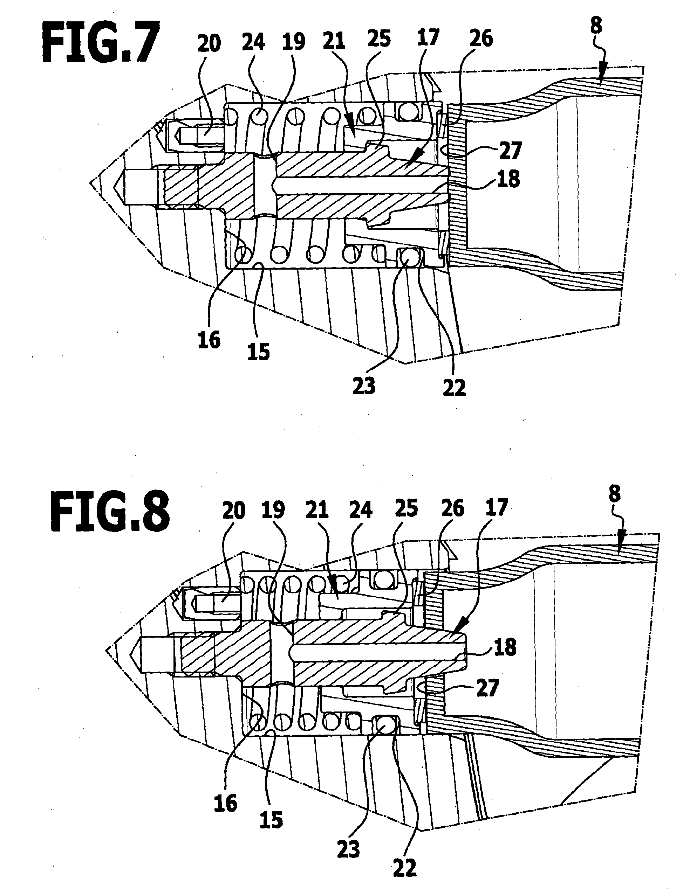

- Figur 7:: eine Längsschnittansicht durch den Anschlußbereich der Druckgaspatrone an einem instrumentenfesten Rohrstutzen bei Beginn des Öffnungsvorganges und
- Figur 8:: eine Ansicht ähnlich Figur 7 mit der in der Anschlußposition angeordneten und geöffneten Druckgaspatrone.

Die Erfindung kann bei chirurgischen Instrumenten der unterschiedlichsten Bauweise eingesetzt werden, sofern diese Teile enthalten, die bewegt werden müssen und die die dazu notwendigen Bewegungskräfte durch ein Druckgas erzeugen. Als Beispiel derartiger Instrumente sind zu nennen Zangen, Scheren oder Greifinstrumente, es kann sich dabei aber auch um druckgasbetätigte Haltearme handeln, bei denen mittels eines Druckgases die Relativbewegungen von verschiedenen Abschnitten eines Haltearmes gegeneinander blockiert bzw. freigegeben werden. In diesem Falle dienen als Werkzeug die Blockiervorrichtungen für die einzelnen Abschnitte des Haltearmes.

Nachstehend wird die Erfindung erläutert am Beispiel eines chirurgischen Werkzeuges, von dem nur ein Griffteil 1 dargestellt ist. An dieses Griffteil 1 können sich Werkzeuge der unterschiedlichsten Art anschließen, beispielsweise zangen- oder scherenförmige Werkzeuge, Greifarme etc.

Diese Instrumente werden üblicherweise über ein in der Zeichnung nicht dargestelltes Zwischenstück, beispielsweise einen Schaft, mit dem Griffteil 1 verbunden. Das Griffteil 1 weist einen verschwenkbaren Auslösegriff 2 auf, mit dem die Betätigung der Werkzeuge gesteuert wird, beispielsweise kann ein solcher Auslösegriff 2 ein in der Zeichnung ebenfalls nicht dargestelltes Ventil für das Einströmen eines Druckgases in eine Arbeitskammer öffnen, in der ein Kolben unter der Wirkung des Druckgases verschoben wird und diese Verschiebebewegung dann über geeignete Übertragungselemente an des Werkzeug überträgt.

Das Griffteil 1 weist neben dem verschwenkbaren Auslösegriff 2 einen festen Griff 3 auf, der bogenförmig ausgebildet ist und bei dem dargestellten Ausführungsbeispiel mit dem verschwenkbaren Auslösegriff 2 gemeinsam eine ringförmige Anordnung ausbildet, die von einem Benutzer umgriffen werden kann.

In dem feststehenden Griff 3 ist in dessen rückwärtigem Bereich, der dem schwenkbaren Griff 3 gegenüberliegt, eine seitlich offene, sich im wesentlichen über die gesamte Länge des Griffes 3 erstreckende Kammer 4 angeordnet, die von einem Deckel 5 verschlossen werden kann, der um eine quer zur Längsrichtung des Griffes 3 verlaufende Achse 6 schwenkbar am Griff 3 gelagert ist. Dieser Deckel 5 bildet einen Teil der Außenkontur des Griffes 3 aus und ist ebenso wie der Griff 3 ergonomisch geformt.

In der von dem Deckel 5 auf der Rückseite des Griffteiles 1 verschlossenen Kammer 4 ist eine hülsenförmige Aufnahme 7 für eine zylindrische Druckgaspatrone 8 angeordnet. Die Aufnahme 7 ist dabei kürzer ausgebildet als die Druckgaspatrone 8, so daß eine in den Innenraum der Aufnahme 7 eingesteckte Druckgaspatrone 8 aus dieser hervorsteht. Dabei ist der Rand der Aufnahme 7 schräg ausgebildet, so daß auf der unteren Seite der Kammer 4 die hülsenförmige Aufnahme 7 länger ist als an der Oberseite. Der Innendurchmesser der von der Aufnahme 7 ausgebildeten Einstecköffnung entspricht dem Außendurchmesser der Druckgaspatrone 8, so daß diese seitlich geführt in der Aufnahme 7 angeordnet ist und lediglich in Längsrichtung der Aufnahme 7 in dieser verschiebbar ist. Der Boden der von der Aufnahme 7 gebildeten Einstecköffnung ist verschlossen, so daß die Einschubtiefe der Druckgaspatrone 8 durch diesen Boden definiert wird.

Die Aufnahme 7 ist in der Kammer 4 in Längsrichtung der Kammer verschieblich und um eine quer zur Längsrichtung der Kammer 4 verlaufende Achse verschwenkbar gelagert. Zu diesem Zweck sind in den Seitenwänden der Kammer 4 auf gegenüberliegenden Seiten zwei schlüssellochförmige Ausnehmungen 9 angeordnet, die gleich ausgebildet sind. Daher wird nachstehend nur eine der beiden Ausnehmungen beschrieben. Die Ausnehmung 9 weist zwei parallel zueinander verlaufende Führungsbahnen 10, 11 auf, die ein Langloch in den Seitenwänden der Kammer 4 ausbilden. An ihrer dem unteren Ende des Griffes 3 und damit der Lagerung des verschwenkbaren Deckels 5 zugewandten Seite enden die parallelen Führungsbahnen 10, 11 in einer kreisförmigen Lageröffnung 12, deren Durchmesser größer ist als der Abstand der beiden Führungsbahnen 10, 11 voneinander.

An der hülsenförmigen Aufnahme 7 sind seitlich längliche Führungselemente 13 angeordnet, die in die schlüssellochförmige Ausnehmung 9 hineinragen. Die Abmessungen der Führungselemente 13 sind dabei so gewählt, daß sie in Längsrichtung dem Durchmesser der Lageröffnung 12 entsprechen, in Querrichtung dagegen dem Abstand der Führungsbahnen 10, 11. Dies führt dazu, daß das Führungselement 13 zwischen den Führungsbahnen 10, 11 nur in einer ganz bestimmten Winkellage eintauchen kann, wenn nämlich das Führungselement 13 parallel zu den Führungsbahnen 10, 11 verläuft. In der Lageröffnung 12 dagegen kann das Führungselement 13 sich verdrehen und unterschiedliche Winkellagen einnehmen, allerdings kann das Führungselement 13 aus der Lageröffnung 12 nur austreten, wenn das Führungselement genau parallel zu den Führungsbahnen 10, 11 steht und in diese verschoben werden kann.

Dadurch ergibt sich eine Schwenklagerung der Aufnahme 7, solange die Führungselemente 13 in die Lageröffnungen 12 eintauchen, dagegen wird die Aufnahme 7 in den Ausnehmungen 9 längsverschieblich gelagert, sobald die Führungselemente 13 zwischen die Führungsbahnen 10, 11 eintauchen, dabei steht die Aufnahme 7 parallel zu den Führungsbahnen 10, 11 und taucht vollständig in die Kammer 4 ein, d.h. bei der Längsverschiebung wird die Aufnahme 7 exakt in Längsrichtung der Kammer 4 verschoben und von dieser aufgenommen.

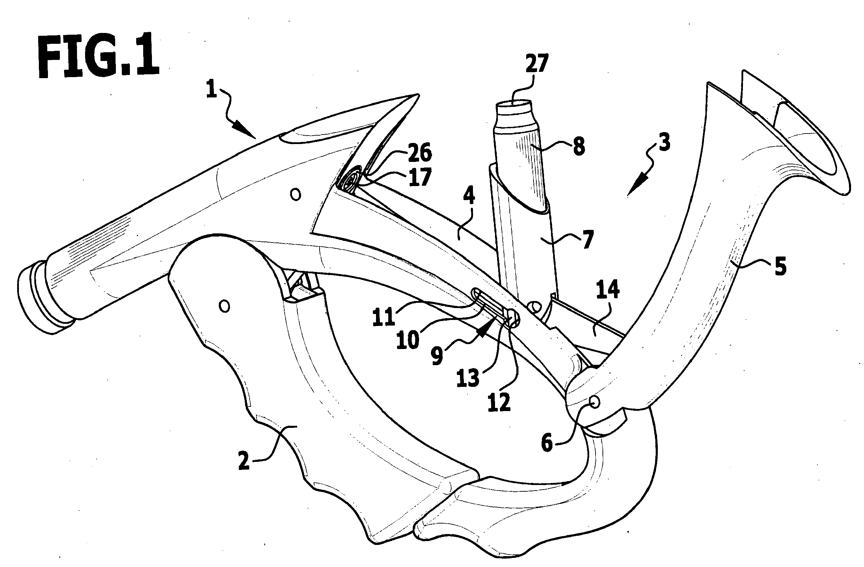

Zur Bewegung der Aufnahme 7 ist diese über einen Hebel 14, der gelenkig an der Aufnahme 7 angreift, mit dem verschwenkbaren Deckel 5 verbunden. Der Hebel 14 ist in relativ geringem Abstand von der Achse 6, um die der Deckel 5 am Griff 3 verschwenkbar ist, gelenkig am Deckel 5 angelenkt, so daß der verschwenkbare Deckel 5 einerseits und der Hebel 14 andererseits gemeinsam eine Kniehebelanordnung ausbilden. Der Hebel 14 greift an der Aufnahme 7 seitlich versetzt zur Mitte der Lageröffnung 12 an, so daß bei einem Vorschieben und Zurückziehen des Hebels 14 durch eine Verschwenkung des Deckels 5 ein Drehmoment auf die Aufnahme 7 ausgeübt wird, solange die Führungselemente 13 in die Lageröffnung 12 eintauchen, d.h. die Verschwenkung des Deckels 5 führt dann zu einer Verschwenkung der Aufnahme 7. Bei vollständig geöffnetem Deckel 5 wird die Aufnahme 7 maximal aus der Kammer 4 ausgeschwenkt und erreicht eine Einsetzposition, in der die Aufnahme 7 für das Auswechseln der Druckgaspatronen 8 in einer besonders günstigen Position steht (Figur 1). Beim Schließen des Deckels 5 wird die Aufnahme 7 sukzessive in die Kammer 4 eingeschwenkt, bis das Führungselement 13 parallel zu den Führungsbahnen 10, 11 angeordnet ist und zwischen diese eintritt. Dadurch erfährt die Aufnahme 7 eine Längsführung, die eine weitere Verschwenkung verhindert, und der Hebel 14 schiebt nunmehr die Aufnahme 7 beim weiteren Schließen des Deckels 5 in Richtung der Führungsbahnen 10, 11 gegen den oberen Teil der Kammer 4.

An diesem oberen Teil der Kammer 4 befindet sich eine koaxial zur Verschieberichtung der Druckgaspatrone 8 angeordnete, zylindrische Druckkammer 15, die zur Kammer 4 hin offen ist und auf der gegenüberliegenden Seite durch einen Boden 16 verschlossen ist. In diesen Boden 16 ist zentral ein Rohrstutzen 17 eingeschraubt, der an seinem der Kammer 4 zugewandten Ende konisch verjüngt ist und somit dornförmig ausgebildet ist. Der Rohrstutzen 17 weist einen in Längsrichtung verlaufenden, zentralen Strömungskanal 18 auf, der von seinem kammerseitigen Ende zu einem Querkanal 19 führt, der den Rohrstutzen 17 diametral durchsetzt und somit eine Verbindung zwischen dem Strömungskanal 18 und der Druckkammer 15 herstellt.

Seitlich neben dem Rohrstutzen 17 ist im Boden 16 ein Auslaß 20 angeordnet, der in aus der Zeichnung nicht ersichtlicher Weise über Strömungskanäle mit dem Werkzeug oder mit Bauteilen des Instrumentes in Verbindung steht, die durch Druckgas angetrieben werden.

Der Rohrstutzen 17 wird konzentrisch umgeben von einer Manschette 21, die auf dem Rohrstutzen 17 längsverschieblich gelagert ist und die in einer Umfangsnut eine Ringdichtung 23 trägt und dadurch gegenüber der Innenwand der Druckkammer 15 abgedichtet ist. Die Manschette 21 ist in Längsrichtung der Druckkammer 15 in dieser verschiebbar und wird dabei einerseits durch den innenliegenden Rohrstutzen 17 und andererseits durch die außenliegende Innenwand der Druckkammer 15 geführt.

An der Manschette 21 stützt sich eine in der Druckkammer 15 angeordnete, den Rohrstutzen 17 konzentrisch umgebende Schraubenfeder 24 ab, deren anderes Ende auf dem Boden 16 der Druckkammer 15 ruht. Gegen die Wirkung der Schraubenfeder 24 kann die Manschette 21 in die Druckkammer 15 eingeschoben werden, die umgekehrte Bewegung unter dem Einfluß der sich entspannenden Schraubenfeder 24 wird durch eine Ringschulter 25 auf der Außenseite des Rohrstutzens 17 begrenzt, an der die Manschette 21 anschlägt.

Die Manschette 21 trägt auf ihrer der Druckgaspatrone 8 zugewandten Stirnseite eine Ringdichtung 26, die den Rohrstutzen 17 konzentrisch im Abstand umgibt und in axialer Richtung den Rohrstutzen 17 geringfügig überragt, wenn die Manschette 21 in ihrer Endstellung an der Ringschulter 25 anliegt.

Nach dem Einlegen der Druckgaspatrone 8 in die Aufnahme 7 wird diese mit Hilfe des verschwenkbaren Deckels 5 in die Verschieberichtung in die Kammer 4 hinein verschwenkt und anschließend in der Kammer 4 in der beschriebenen Weise in Richtung auf den Rohrstutzen 17 verschoben. Dabei legt sich die Stirnseite der noch verschlossenen Druckgaspatrone 8 zunächst an die Ringdichtung 26 an, wobei durch die Schraubenfeder 24 Toleranzen ausgeglichen werden, die sich bei den Abmessungen der Druckgaspatrone 8 und der Aufnahme 7 ergeben könnten. In jedem Fall erfolgt eine Abdichtung des innerhalb der Ringdichtung 26 liegenden Raumes unter der Einwirkung der die Ringdichtung 26 gegen die Druckgaspatrone 8 drückenden Schraubenfeder 24, bevor der Rohrstutzen 17 die Druckgaspatrone 8 berührt.

Beim weiteren Vorschieben der Druckgaspatrone 8 gegen den Rohrstutzen 17 durchdringt dieser die Oberseite 27 der Druckgaspatrone 8 und tritt in diese ein, so daß der Innenraum der Druckgaspatrone 8 über den Strömungskanal 18 und den Querkanal 19 mit dem Inneren der Druckkammer 15 in Verbindung gelangt. Solange der Auslaß 20 vor der Betätigung des chirurgischen Instruments geschlossen ist, baut sich dadurch in der Druckkammer 15 ein hoher Druck auf, der dazu führt, daß die Manschette 21 mit einer zusätzlichen Kraft gegen die Oberseite 27 der Druckgaspatrone 8 gedrückt wird. Dies liegt daran, daß die Manschette 21 zwar beidseitig von dem Druckgas beaufschlagt wird, daß aber die von dem Druckgas beaufschlagten Flächen auf gegenüberliegenden Seiten der Manschette unterschiedlich sind. Auf der Seite der Druckgaspatrone 8 ist diese wirksame Fläche die Fläche innerhalb der Ringdichtung 26, auf der gegenüberliegenden Seite wird diese Fläche durch die ringförmige Querschnittsfläche der Druckkammer 15 abzüglich der Querschnittsfläche des Rohrstutzens 17 bestimmt, und diese Fläche ist wesentlich größer als die von der Ringdichtung 26 eingeschlossene Fläche. Dies führt dazu, daß durch das Druckgas die Manschette 21 mit einer die Kraft der Schraubenfeder 24 verstärkenden Kraft gegen die Oberseite 27 der Druckgaspatrone 8 gedrückt wird, und dadurch wird die Dichtwirkung ganz erheblich erhöht.

Mit abnehmendem Druck des Druckgases wird zwar diese zusätzliche Kraft geringer, mit der die Ringdichtung 26 gegen die Oberseite 27 gedrückt wird, dann ist aber auch die Druckdifferenz zum Umgebungsdruck geringer, so daß die Dichtung trotzdem zuverlässig ausreicht.

Andererseits ist die Dichtkraft durch die Unterstützung des Druckgases so erhöht, daß ein erheblicher Teil der Dichtkraft durch diesen Effekt aufgebracht wird, die von der Schraubenfeder 24 aufgebrachte Dichtkraft muß also nicht so groß sein, daß sie alleine zur zuverlässigen Abdichtung ausreichen würde. Dementsprechend kann die Schraubenfeder 24 relativ klein gewählt werden, so daß sich keine Erhöhung der Abmessungen des Griffteiles ergibt.

Nach Entleerung der Druckgaspatrone 8 wird die Aufnahme 7 durch Ausschwenken des Deckels 5 freigegeben, d.h. die Aufnahme 7 wird dadurch zurückgezogen, und die Schraubenfeder 24 verschiebt die Druckgaspatrone 8 zusammen mit der Aufnahme 7, d.h. die Schraubenfeder 24 hat auch noch den Effekt einer Rückholfeder, die die Druckgaspatrone 8 von dem Rohrstutzen 17 abschiebt, sobald die Aufnahme 7 mit Hilfe des verschwenkbaren Deckels 5 aus der Anschlußposition zurückgefahren wird.

## Patentansprüche

1. Chirurgisches Instrument mit einem druckgasbetätigten Werkzeug und mit einer Aufnahme (7) für eine Druckgaspatrone (8) sowie einem druckdichten Anschluss zur Verbindung der Druckgaspatrone (8) mit dem Werkzeug, wobei die Aufnahme (7) in eine Anschlussposition bewegbar ist, in der die Druckgaspatrone (8) derart gegen einen am Instrument angeordneten, einen Strömungskanal (18) aufweisenden, dornförmigen Rohrstutzen (17) drückbar ist, dass dieser die Druckgaspatrone (8) ansticht und öffnet und den Innenraum der Druckgaspatrone (8) mit einer zu dem Werkzeug führenden Strömungsleitung (20) für das Druckgas verbindet und wobei eine den Rohrstutzen (17) umgebende, an der Druckgaspatrone (8) anlegbare Ringdichtung (26) vorgesehen ist, die die Druckgaspatrone (8) gegenüber dem Instrument abdichtet, **dadurch gekennzeichnet, dass** die Ringdichtung (26) durch eine Feder (24) gegen die Druckgaspatrone (8) gedrückt wird.

2. Instrument nach Anspruch 1, **dadurch gekennzeichnet, dass** der Verschiebeweg der Ringdichtung (26) in Kraftrichtung der Feder (24) durch einen Anschlag (25) begrenzt ist.

3. Instrument nach einem der Ansprüche 1 oder 2, **dadurch gekennzeichnet, dass** die Ringdichtung (26) bei entspannter Feder (24) über den Rohrstutzen (17) vorsteht.

4. Instrument nach einem der voranstehenden Ansprüche, **dadurch gekennzeichnet, dass** die Ringdichtung (26) an einer den Rohrstutzen (17) umgebenden Manschette (21) angeordnet ist.

5. Instrument nach Anspruch 4, **dadurch gekennzeichnet, dass** die Manschette (21) durch eine Feder (24) gegen die Druckgaspatrone (8) gedrückt wird.

6. Instrument nach Anspruch 5, **dadurch gekennzeichnet, dass** der Verschiebeweg der Manschette (21) in Kraftrichtung der Feder (24) durch einen Anschlag (25) begrenzt ist.

7. Instrument nach Anspruch 6, **dadurch gekennzeichnet, dass** der Anschlag (25) am Rohrstutzen (17) angeordnet ist.

8. Instrument nach einem der Ansprüche 4 bis 7, **dadurch gekennzeichnet, dass** der Rohrstutzen (17) und die ihn umgebende Manschette (21) in einer Druckkammer (15) angeordnet sind und dass die Manschette (21) in der Druckkammer (15) in Längsrichtung des Rohrstutzens (17) mittels einer Dichtung (23) abgedichtet verschieblich gelagert ist.

9. Instrument nach Anspruch 8, **dadurch gekennzeichnet, dass** der Strömungskanal (18) des Rohrstutzens (17) auf der der Druckgaspatrone (8) abgewandten Seite der Dichtung (23) in die Druckkammer (15) einmündet und dass die Druckkammer (15) einen zu dem druckgasbetätigten - Werkzeug führenden Auslaß (20) aufweist.

10. Instrument nach Anspruch 9, **dadurch gekennzeichnet, dass** die Querschnittsfläche der Manschette (21) größer ist als die von der Ringdichtung (26) zwischen Manschette (21) und Druckgaspatrone (8) umschlossene Fläche.

## Claims

1. A surgical instrument having a tool actuated by compressed gas and having a receiver (7) for a compressed-gas cartridge (8) as well as a pressure-tight connection for connection of the compressed-gas cartridge (8) to the tool, wherein the receiver (7) is movable into a connecting position in which the compressed-gas cartridge (8) can be pushed against a spike-shaped tubular connection piece (17), arranged on the instrument and having a flow channel (18), in such a manner that the tubular connection piece (17) punctures and opens the compressed-gas cartridge (8) and connects the interior of the compressed-gas cartridge (8) to a flow line (20), for the compressed gas, leading to the tool, and wherein an annular seal (26), surrounding the tubular connection piece (17) and able to be placed against the compressed-gas cartridge (8), is provided and seals the compressed-gas cartridge (8) with respect to the instrument, **characterised in that** the annular seal (26) is pressed against the compressed-gas cartridge (8) by a spring (24).

2. An instrument according to claim 1, **characterised in that** the displacement path of the annular seal (26) is bounded by a stop (25) in the direction of force of the spring (24).

3. An instrument according to one of claims 1 and 2, **characterised in that** the annular seal (26) projects beyond the tubular connection piece (17) when the spring (24) is relaxed.

4. An instrument according to any one of the preceding claims, **characterised in that** the annular seal (26) is arranged on a collar (21) surrounding the tubular connection piece (17).

5. An instrument according to claim 4, **characterised in that** the collar (21) is pushed against the compressed-gas cartridge (8) by a spring (24).

6. An instrument according to claim 5, **characterised in that** the displacement path of the collar (21) is bounded by a stop (25) in the direction of force of the spring (24).

7. An instrument according to claim 6, **characterised in that** the stop (25) is arranged on the tubular connection piece (17).

8. An instrument according to any one of claims 4 to 7, **characterised in that** the tubular connection piece (17) and the collar (21) surrounding it are arranged in a pressure chamber (15), and **in that** the collar (21) is mounted in the pressure chamber (15) so as to be displaceable in the longitudinal direction of the tubular connection piece (17) in a manner sealed by means of a seal (23).

9. An instrument according to claim 8, **characterised in that** the flow channel (18) of the tubular connection piece (17) flows into the pressure chamber (15) on that side of the seal (23) which is remote from the compressed-gas cartridge (8), and **in that** the pressure chamber (15) has an outlet (20) leading to the tool actuated by compressed gas.

10. An instrument according to claim 9, **characterised in that** the cross-sectional area of the collar (21) is greater than the area enclosed by the annular seal (26) between the collar (21) and the compressed-gas cartridge (8).

## Revendications

1. Instrument chirurgical comprenant un outil actionné par un gaz sous pression, et un logement de réception (7) pour une cartouche de gaz sous pression (8), ainsi qu'un raccord étanche à la pression pour relier la cartouche de gaz sous pression (8) à l'outil, instrument dans lequel le logement de réception (7) peut être déplacé dans une position de raccordement dans laquelle la cartouche de gaz sous pression (8) peut être pressée contre un embout tubulaire (17) en forme de poinçon agencé sur l'instrument et présentant un canal d'écoulement (18), de façon telle que cet embout tubulaire transperce et ouvre la cartouche de gaz sous pression (8) et relie la chambre intérieure de la cartouche de gaz sous pression (8) à une conduite d'écoulement (20) pour le gaz sous pression menant à l'outil, et dans l'instrument est prévu un joint d'étanchéité annulaire (26) qui entoure l'embout tubulaire (17) et peut être appliqué contre la cartouche de gaz sous pression (8) en assurant l'étanchéité de la cartouche de gaz sous pression (8) par rapport à l'instrument, **caractérisé en ce que** le joint d'étanchéité annulaire (26) est pressé contre la cartouche de gaz sous pression (8) par un ressort (24).

2. Instrument selon la revendication 1, **caractérisé en ce que** la course de déplacement ou de coulissement du joint d'étanchéité annulaire (26) dans la direction de la force du ressort (24), est limitée par une butée (25).

3. Instrument selon l'une des revendications 1 ou 2, **caractérisé en ce que** le joint d'étanchéité annulaire (26) fait saillie au-devant de l'embout tubulaire (17) lorsque le ressort (24) est détendu.

4. Instrument selon l'une des revendications précédentes, **caractérisé en ce que** le joint d'étanchéité annulaire (26) est agencé sur un manchon (21) qui entoure l'embout tubulaire (17).

5. Instrument selon la revendication 4, **caractérisé en ce que** le manchon (21) est pressé par un ressort (24) contre la cartouche de gaz sous pression (8).

6. Instrument selon la revendication 5, **caractérisé en ce que** la course de déplacement ou de coulissement du manchon (21) dans la direction de la force du ressort (24) est limitée par une butée (25).

7. Instrument selon la revendication 6, **caractérisé en ce que** la butée (25) est agencée sur l'embout tubulaire (17).

8. Instrument selon l'une des revendications 4 à 7, **caractérisé en ce que** l'embout tubulaire (17) et le manchon (21) qui l'entoure sont agencés dans une chambre de pression (15), et **en ce que** le manchon (21) est monté dans la chambre de pression (15) en pouvant y coulisser dans la direction longitudinale de l'embout tubulaire (17) et en étant rendu étanche au moyen d'un joint d'étanchéité (23).

9. Instrument selon la revendication 8, **caractérisé en ce que** le canal d'écoulement (18) de l'embout tubulaire (17) débouche dans la chambre de pression (15), sur le côté du joint d'étanchéité (23) opposé au côté où se trouve la cartouche de gaz sous pression (8), et **en ce que** la chambre de pression (15) présente une sortie (20) menant à l'outil actionné par le gaz sous pression.

10. Instrument selon la revendication 9, **caractérisé en ce que** la surface de la section transversale du manchon (21) est plus grande que la surface entourée par le joint d'étanchéité annulaire (26) entre le manchon (21) et la cartouche de gaz sous pression (8).
